(19) Europäisches Patentamt
European Patent Office
Office européen des brevets

(11) Numéro de publication: 0 382 934 B1

(12) # FASCICULE DE BREVET EUROPEEN

(45) Date de publication de fascicule du brevet: **28.04.93**

(51) Int. Cl.$^5$: **C07C 49/453**, C07C 49/643, C07C 45/62, C07C 45/72, A61K 7/00

(21) Numéro de dépôt: **89123373.6**

(22) Date de dépôt: **18.12.89**

(54) **Cétones tricycliques, procédé pour leur préparation et leur utilisation en tant qu'agents parfumants.**

(30) Priorité: **14.02.89 CH 511/89**

(43) Date de publication de la demande:
**22.08.90 Bulletin 90/34**

(45) Mention de la délivrance du brevet:
**28.04.93 Bulletin 93/17**

(84) Etats contractants désignés:
**CH DE FR GB LI NL**

(56) Documents cités:
**EP-A- 0 074 693**
**EP-A- 0 208 994**
**DE-A- 2 901 119**
**DE-B- 2 461 593**

**TETRAHEDRON LETTERS, no. 12, 1976, pages 907-910, Pergamon Press, Oxford, GB; W. HIMMELE et al, "Hydroformylierung von alpha-PINEN"**

(73) Titulaire: **FIRMENICH SA**
**1, route des Jeunes**
**CH-1211 Genève 8(CH)**

(72) Inventeur: **Giersch, Wolfgang**
**323, rue de Bernex**
**CH-1233 Bernex(CH)**
Inventeur: **Schulte-Elte, Karl-Heinrich**
**44, chemin de Carabot**
**CH-1213 Onex(CH)**

(74) Mandataire: **Salvadori, Giuseppe, Dr.**
**c/o Firmenich S.A. Case Postale 239**
**CH-1211 Genève 8 (CH)**

EP 0 382 934 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait au domaine de la parfumerie. Elle concerne plus particulièrement des cétones tricycliques de formule

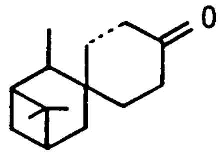

(I)

pouvant posséder une liaison simple ou double dans la position indiquée par les pointillés. Il s'agit de dérivés du pinène, composé qui est employé en tant que produit de départ pour leur synthèse.

C'est précisément en fonction de la configuration spécifique du pinène que l'on obtient les cétones (I) sous forme d'un mélange racémate ou sous forme d'énantiomères optiquement actifs.

La présente invention a comme objet tout particulièrement les composés de formule

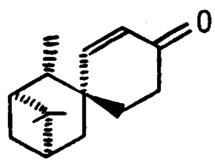

et

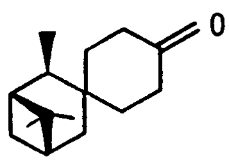

(Ia) (Ib)

Il s'est en effet avéré que ces deux composés, définis respectivement comme (+)-(1S,2S,3S)-2,6,6-triméthyl-bicyclo[3.1.1]heptane-3-spiro-1'-(2'-cyclo-hexén-4'-one) et (+)-(1R,2R)-2,6,6-triméthyl-bicyclo-[3.1.1]heptane-3-spiro-1'-(4'-cyclohexanone) pouvaient développer des notes odorantes particulièrement intéressantes et trouvaient de ce fait une utilisation avantageuse dans le domaine de la parfumerie. Leur caractère olfactif peut être défini comme étant de type boisé. Leur note est bien distincte, chaude avec un côté épicé qui rappelle certains aspects de la coumarine. Leur puissance est prononcée et leur ténacité ainsi que leur substantivité sont tout à fait remarquables tant sur la peau que sur des substrats variés, les textiles en particulier.

Au vu de leurs propriétés, les composés de l'invention peuvent être employés pour la manufacture de parfums et bases parfumantes ou pour le parfumage de produits de consommation variés, tels les savons, les cosmétiques, les shampoings, les détergents, les produits d'entretien ou les désodorisants corporels ou d'air ambiant. Les composés de l'invention trouvent également une utilisation dans le parfumage de matériaux polymériques, tels ceux qui sont communément utilisés à titre de support pour des dispositifs désodorisants ou assainissants d'air ambiant ou d'enceintes fermées.

Les proportions dans lesquelles les composés de l'invention peuvent developper les caractères odorants désirés peuvent varier dans une gamme de valeurs très étendue. L'homme de l'art sait par expérience que de telles valeurs dépendent de l'effet particulier recherché ainsi, bien entendu, que de la nature des produits que l'on désire parfumer. On sait également que ces proportions sont fonction de la nature des autres constituants dans une composition donnée lorsque les composés de formule (I) sont utilisés en tant qu'ingrédients dans une base parfumante ou dans un concentré en mélange avec d'autres ingrédients, des dissolvants ou des adjuvants usuels. Par autre ingrédient il faut entendre tout composé d'origine naturelle ou synthétique dont l'emploi en parfumerie est courant ou tout au moins documenté. Il peut s'agir de composés appartenant à des classes chimiques variées telles les cétones, les aldéhydes, les alcools ou les esters en particulier. La seule limitation dans le choix que le parfumeur doit opérer parmi de tels produits est constituée par l'équilibre olfactif de la composition résultante.

Compte tenu de ce qui précède, les composés de l'invention peuvent, par exemple, être employés dans des proportions de l'ordre de 5 à 10%, voire 20% en poids par rapport au poids total de la composition dans laquelle ils sont incorporés. Des valeurs inférieures sont utilisées lors du parfumage de produits tels les savons ou les détergents pour lesquels des effets intéressants peuvent déjà être obtenus par l'adjonction des composés (I) dans des proportions de l'ordre de 0,1 à 0,5%, voire 1% en poids.

Les composés de formule (I) sont des entités chimiques nouvelles. Comme il a été indiqué plus haut, lesdits composés sont obtenus par un procédé original à partir de (-)-alpha-pinène à travers la formation de

(+)-3-formyl-pinane selon la méthode décrite par W. Himmele et H. Siegel [Tetrah. Lett. 907-10(1976)], lequel produit donne par addition avec de la méthyl-vinyl cétone la (+)-(1S,2S,3S)-2,6,6-triméthyl-bicyclo-[3.1.1]heptane-3-spiro-1′-(2′-cyclohexén-4′-one).

Lorsqu'on utilise comme produit de départ le (+)-alpha-pinène à la place de son énantiomère (-), on obtient selon les auteurs précités le (-)-3-formyl-pinane qui, par addition de méthyl-vinyl cétone, fournit la (-)-(1R,2R,3R)-2,6,6-triméthyl-bicyclo[3.1.1]heptane-3-spiro-1′-(2′-cyclohexén-4′-one), laquelle cétone donne par hydrogénation catalytique la (+)-(1R,2R)-2,6,6-triméthyl-bicyclo[3.1.1]heptane-3-spiro-1′-(4′-cyclohexanone).

La présente invention a donc également pour objet un procédé pour la préparation des composés de formule (I), lequel procédé est caractérisé en ce que

a. on additionne à du (+)-3-formyl-pinane de la méthyl-vinyl cétone pour fournir la (+)-(1S,2S,3S)-2,6,6-triméthyl-bicyclo[3.1.1]heptane-3-spiro-1′-(2′-cyclo-hexén-4′-one) de formule (Ia), ou

a′. on additionne à du (-)-3-formyl-pinane de la méthyl-vinyl cétone pour fournir la (-)-(1R,2R,3R)-2,6,6-triméthyl-bicyclo[3.1.1]heptane-3-spiro-1′-(2′-cyclohexén-4′-one), et

b. on soumet le composé ainsi obtenu à une hydrogénation catalytique pour donner la (+)-(1R,2R)-2,6,6-triméthyl-bicyclo[3.1.1]heptane-3-spiro-1′-(4′-cyclohexanone) de formule (Ib).

Le procédé sus-mentionné peut être illustré à l'aide du schéma réactionnel suivant:

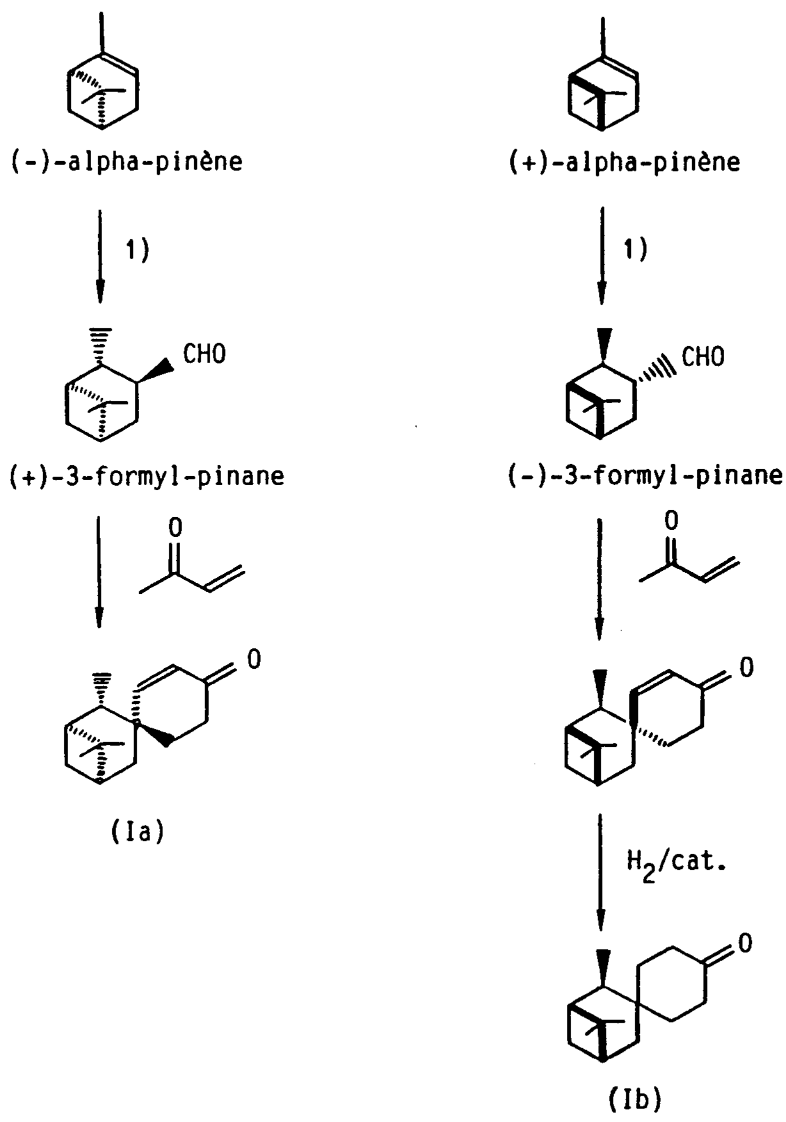


(-)-alpha-pinène (+)-alpha-pinène

(+)-3-formyl-pinane (-)-3-formyl-pinane

(Ia)

(Ib)

1) cf. Tetrah. Lett. 907-10 (1976)

L'addition de méthyl-vinyl cétone sur le (+)-, respectivement (-)-3-formyl-pinane s'effectue dans des conditions de réaction courantes. De préférence, les deux réactifs en solution alcoolique sont chauffés après addition à la température d'ébullition ou à une température proche de celle-ci. Le produit d'addition résultant peut être séparé du mélange réactionnel par simple distillation.

L'étape b) du procédé, qui consiste en la réduction par hydrogénation catalytique, est effectuée en soumettant la cétone résultant de l'étape a') à l'action de l'hydrogène dans un réacteur à pression atmosphérique en présence d'un catalyseur métallique usuel, par exemple le palladium, de préférence sur charbon actif, ou le $PtO_2$.

La cétone saturée désirée (Ib) peut être isolée du milieu réactionnel par les traitements habituels de filtration, concentration et distulation. La méthode particulière suivie sera décrite dans le détail dans les exemples spéci£iques donnés ci-après. Dans lesdits exemples, les températures sont indiquées en degrés centigrades et les abréviations ont le sens usuel dans l'art.

Exemple 1

Préparation de (+)-(1S,2S,3S)-2,6,6-triméthyl-bicyclo[3.1.1]heptane-3-spiro-1′-(2′-cyclohexén-4′-one)

83,0 g (0,5 moles) de (+)-3-formyl-pinane [origine: BASF, Ludwigshafen/Rhein (RFA)] ayant [alpha]-$^{20}{}_D$ = +19,7° ont été mélangés avec 63 g (0,9 moles) de méthylvinyl cétone, dans 500 ml d'éthanol, en présence de 1 g de KOH, dans un réacteur comportant un réfrigérant et un agitateur. Le mélange résultant a été ensuite chauffé à reflux pendant 6 h. Après refroidissement, on a concentré le tout sous pression réduite et on a repris le résidu avec de l'éther de pétrole 30/50°, puis on a lavé avec 3 fractions d'eau. Après élimination du solvant, on a distillé sous vide et obtenu 82,6 g (rend. 76%) d'une fraction ayant eb. 90-120°/13,3 Pa.

Ses caractères analytiques étaient les suivants:
[alpha]$^{20}{}_D$ = +28,7°
IR : 1680 $cm^{-1}$;
$^1$H-RMN : 1,01(3H,s); 1,05(3H,d,J=7Hz); 1,25(3H,s); 5,92 et 7,14(2H,2d,J=11Hz) delta ppm.

Exemple 2

Préparation de (+)-(1R,2R)-2,6,6-triméthyl-bicyclo[3.1.1]heptane-3-spiro-1′-(4′-cyclohexanone)

a′) 16,6 g de (-)-3-formyl-pinane ont été mélangés avec 12,6 g de méthyl-vinyl cétone dans 100 ml d'éthanol en présence de 1 g de KOH et le mélange résultant a été chauffé à reflux pendant 6 h. Après évaporation de l'éthanol, le résidu qui en a résulté a été repris avec de l'éther de pétrole 30/50° et ensuite lavé avec de l'eau jusqu'à neutralité. Le solvant a été éliminé par évaporation et la (-)-(1R,2R,3R)-2,6,6-triméthyl-bicyclo[3.1.1]heptane-3-spiro-1′-(2′-cyclohexén-4′-one) a été recueillie à eb. 92-98°/8 Pa; 14,6 g (rend. 67%). [alpha]$^{20}{}_D$ = -69,5°.

b) 3,1 g de la cétone obtenue selon la méthode décrite sous lettre a') ci-dessus, dissous dans de l'acétate d'éthyle, ont été soumis à une hydrogénation catalytique à pression atmosphérique en présence de Pd à 5% sur charbon actif. La réaction a été poursuivie jusqu'à ce que l'absorption d'hydrogène s'arrête. Les traitements usuels de filtration, concentration et distillation ont fourni la cétone désirée à eb. 120° (temp. du bain)/13,3 Pa; 3 g; F. 87-9°.
[alpha]$^{20}{}_D$ = +14,7° ($CHCl_3$).
IR: 1700 $cm^{-1}$;
$^1$H-RMN: 1,0(3H,s); 1,07(3H,d,J=7Hz); 1,25(3H,s) delta ppm;
SM : $M^+$ = 220(2); m/e: 221(2), 205(3),) 177(10), 164(13), 136(18), 110(40), 95(47), 83(93), 69(52), 55-(100), 41(100).

Exemple 3

Composition parfumante

Une composition parfumante de base a été préparée en mélangeant les ingrédients suivants (parties en poids):

| | |
|---|---|
| Salicylate de benzyle | 120 |
| Alcool phényléthylique | 100 |
| Hydroxycitronellal | 80 |
| Hédione (marque enregistrée) 1) | 80 |
| gamma-Méthylionone | 80 |
| Essence de bergamote | 70 |
| Essence de rose synth. | 70 |
| Essence de jasmin synth. | 60 |
| Coumarine | 30 |
| Astrotone (marque enregistrée) 2) | 20 |
| Mousse cristal | 20 |
| Musc cétone | 20 |
| Laevocitrol 3) | 20 |
| Polysantol (marque enregistrée) 4) à 10%* | 20 |
| Essence de citron "sfumatrice" | 20 |
| Héliopropanal | 10 |
| Oxyde de rose à 10%* | 10 |
| Essence de petitgrain Bigarade | 10 |
| Propionate de citronellyle | 10 |
| Géranylacétone | 10 |
| Tonalid (marque enregistrée) 5) | 10 |
| Résine de labdanum à 50%* | 5 |
| Aldéhyde undécylénique à 10%* | 5 |
| Total | 880 |

1) Firmenich SA ; dihydrojasmonate de méthyle
2) Rhône-Poulenc ; éthylène brassilate
3) Firmenich SA ; l-citronellol
4) Firmenich SA ; (E)-3,3-diméthyl-5-(2,2,3-triméthyl-3-cyclopentén-1-yl)-4-pentén-2-ol
5) PFW ; 6-acétyl-1,1,3,4,4,6-hexaméthyl-tétrahydronaphtalène

Lorsqu'on ajoute à 88 g de la composition de base ainsi préparée 12 g de (+)-(1S,2S,3S)-2,6,6-triméthyl-bicyclo[3.1.1]heptane-3-spiro-1'-(2'cyclohexén-4'-one), dont l'activité optique était [alpha]-$^{20}{}_{D}$ = +28,7°, on obtient une nouvelle composition ayant un caractère nettement plus riche et boisé. Par dilution de la composition résultante dans l'éthanol à 95% à raison de 20% en poids, on obtient une composition alcoolique dont l'élégance et la richesse sont encore plus prononcées que celles de la composition concentrée.
Des effets analogues, quoique moins prononcés, ont été obtenus par l'emploi dans la même base

parfumante de (+)-(1R,2R)-2,6,6-triméthyl-bicyclo[3.1.1]heptane-3-spiro-1'-(4'-cyclohexanone).

Exemple 4

Parfumage de savon

100 g de savon en copeaux, obtenu à partis d'une base de savon au sodium non parfumé préparée avec de l'huile de coco et de suif, ont été mélangés avec 0,5 g de (+)-(1S,2S,3S)-2,6,6-triméthyl-bicyclo-[3.1.1]heptane-3-spiro-1'-(2'-cyclohexén-4'-one) jusqu'à l'obtention d'une pâte homogène. La composition de savon ainsi obtenue possédait une odeur boisée-épicée. En remplaçant le composé mentionné par le (+)-(1R,2R)-2,6,6-triméthyl-bicyclo[3.1.1]heptane-3-spiro-1'-(4'-cyclohexanone), on observe des effets similaires.

Exemple 5

Parfumage d'un détergent solide

Une base détergente solide a été préparée en mélangeant les ingrédients suivants (parties en poids) :

| | |
|---|---|
| Alkyl-benzène sulphonate lin. de sodium (longueur de la chaîne $C_{11-5}$) | 8,0 |
| Alcool de suif éthoxylé (14EO) | 2,9 |
| Savon au sodium (longueur de la chaîne $C_{12-16}$ 13-26% ; $C_{18-22}$ 74-87%) | 3,5 |
| Triphosphate de sodium | 43,8 |
| Silicate de sodium | 7,5 |
| Silicate de magnésium | 1,9 |
| Carboxyméthylcellulose | 1,2 |
| Sodium EDTA | 0,2 |
| Sulphate de sodium | 21,2 |
| Eau | 9,8 |
| Total | 100,0 |

L'addition à un échantillon de la base détergente de (+)-(1S,2S,3S)-2,6,6-triméthyl-bicyclo[3.1.1]-heptane-3-spiro-1'-(2'-cyclohexén-4'-one) ou de (+)-(1R,2R)-2,6,6-triméthyl-bicyclo[3.1.1]heptane-3-spiro-1'-(4'-cyclohexanone) à raison de 0,05% en poids lui confère un caractère boisé. Un nouvel échantillon de la base détergente a été parfumé à l'aide de la composition suivante à raison de 0,25% :

| | |
|---|---|
| tert-Butyl-cyclohexyl acétate | 400 |
| Hédione (marque enregistrée) [1)2)] | 200 |
| Méthylionone [1)] | 100 |
| Essence de jasmin synth. | 100 |
| Galaxolide (marque enregistrée) [3)] | 100 |
| Ⓐ | 100 |
| Total | 1000 |

1) Firmenich SA
2) Dihydrojasmonate de méthyle
3) IFF : 1,3,4,6,7,8-hexahydro-4,6,6,7,8,8-hexaméthyl-cyclopenta-gamma-2-benzopyranne
Ⓐ = (+)-(1S,2S,3S)-2,6,6-triméthy-bicyclo[3.1.1]heptane-3-spiro-1'-(2'-cyclohexén-4'-one)

## Revendications

1. Composés cétoniques tricycliques de formule

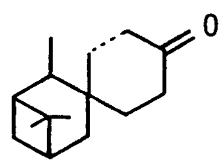

(I)

pouvant contenir une liaison simple ou double dans la position indiquée par les pointillés.

2. A titre de composé selon la revendication 1, la (+)-(1S,2S,3S)-2,6,6-triméthyl-bicyclo[3.1.1]heptane-3-spiro-1′-(2′-cyclohexén-4′-one).

3. A titre de composé selon la revendication 1, la (+)-(1R,2R)-2,6,6-triméthyl-bicyclo[3.1.1]heptane-3-spiro-1′-(4′-cyclohexanone).

4. Utilisation d'un composé cétonique tricyclique selon la revendication 1, à titre d'ingrédient parfumant.

5. Composition parfumante contenant à titre d'ingrédient parfumant actif un composé selon la revendication 1.

6. Produit parfumé contenant à titre d'ingrédient parfumant actif un composé selon la revendication 1.

7. A titre de produit parfumé selon la revendication 6, un détergent, un savon ou un désodorisant d'air ambiant.

8. Procédé pour la préparation des composés de formule (I) selon la revendication 1, caractérisé en ce que
   a. on additionne à du (+)-3-formyl-pinane de la méthyl-vinyl cétone pour fournir la (+)-(1S,2S,3S)-2,6,6-triméthyl-bicyclo[3.1.1]heptane-3-spiro-1′-(2′-cyclohexén-4′-one) de formule (Ia)

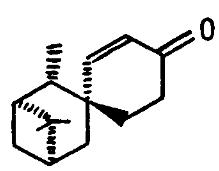

(Ia)

   ou
   a′. on additionne à du (-)-3-formyl-pinane de la méthyl-vinyl cétone pour fournir la (-)-(1R,2R,3R)-2,6,6-triméthyl-bicyclo[3.1.1]heptane-3-spiro-1′-(2′-cyclohexén-4′-one), et
   b. on soumet le composé ainsi obtenu à une hydrogénation catalytique pour donner la (+)-(1R,2R)-2,6,6-triméthyl-bicyclo[3.1.1]heptane-3-spiro-1′-(4′-cyclohexanone) de formule (Ib)

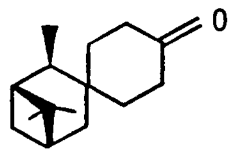

(Ib)

## Claims

1. Tricyclic ketone compounds of formula

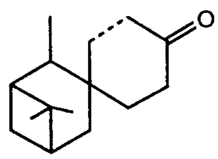

(I)

which can have a single or double bond in the position indicated by the dotted line.

2. (+)-(1S,2S,3S)-2,6,6-Trimethyl-bicyclo[3.1.1]heptane-3-spiro-1'-(2'-cyclohexen-4'-one) as a compound according to claim 1.

3. (+)-(1R,2R)-2,6,6-Trimethyl-bicyclo[3.1.1]heptane-3-spiro-1'-(4'-cyclohexanone) as a compound according to claim 1.

4. Use of a tricyclic ketone compound according to claim 1 as perfuming ingredient.

5. Perfuming composition containing as active perfuming ingredient a compound according to claim 1.

6. Perfumed product containing as active perfuming ingredient a compound according to claim 1.

7. As a perfumed product according to claim 6, a detergent, a soap or an air freshener.

8. Process for the preparation of compounds of formula (I) according to claim 1, characterized in that
   a. methyl vinyl ketone is added to (+)-3-formyl-pinane to yield (+)-(1S,2S,3S)-2,6,6-trimethyl-bicyclo[3.1.1]heptane-3-spiro-1'-(2'-cyclohexen-4'-one) of formula (Ia)

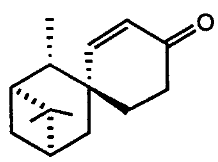

(Ia)

   or
   a'. methyl vinyl ketone is added to (-)-3-formyl-pinane to yield (-)-(1R,2R,3R)-2,6,6-trimethyl-bicyclo-[3.1.1]heptane-3-spiro-1'-(2'-cyclohexen-4'-one), and
   b. the compound thus obtained is subjected to catalytic hydrogenation to yield (+)-(1R,2R)-2,6,6-trimethyl-bicyclo[3.1.1]heptane-3-spiro-1'-(4'-cyclohexanone) having the formula (Ib)

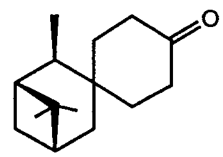

(Ib)

## Patentansprüche

1. Tricyclische Ketone der Formel

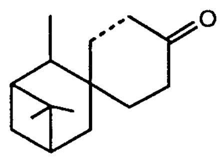

(I),

die in der durch die gestrichelte Linie gekennzeichneten Stellung eine Einzel- oder Doppelbindung enthält.

2. (+)-(1S,2S,3S)-2,6,6-Trimethyl-bicyclo[3.1.1]heptan-3-spiro-1'-(2'-cyclohexen-4'-on) als Verbindung gemäß Anspruch 1.

3. (+)-(1R,2R)-2,6,6-Trimethyl-bicyclo[3.1.1]heptan-3-spiro-1'-(4'-cyclohexanon) als Verbindung gemäß Anspruch 1.

4. Verwendung eines tricyclischen Ketons gemäß Anspruch 1 als Riechstoff.

5. Riechstoffkomposition, die als aktiven Riechstoff eine Verbindung gemäß Anspruch 1 enthält.

6. Parfümiertes Produkt, das als aktiven Riechstoff eine Verbindung gemäß Anspruch 1 enthält.

7. Ein Reinigungsmittel, eine Seife oder ein Raumbedüfter als parfümiertes Produkt gemäß Anspruch 6.

8. Verfahren zur Herstellung von Verbindungen der Formel (I) gemäß Anspruch 1, dadurch gekennzeichnet, daß man
   a. an (+)-3-Formyl-pinan Methylvinylketon addiert, um (+)-(1S,2S,3S)-2,6,6-Trimethyl-bicyclo[3.1.1]-heptan-3-spiro-1'-(2'-cyclohexen-4'-on) der Formel (Ia)

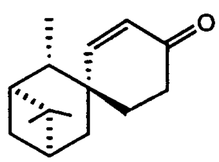

(Ia)

   zu erhalten,
   oder
   a'. an (-)-3-Formyl-pinan Methylvinylketon addiert, um (-)-(1R,2R,3R)-2,6,6-Trimethyl-bicyclo[3.1.1]-heptan-3-spiro-1'-(2'cyclohexen-4'-on) zu erhalten, und

b. die so erhaltene Verbindung einer katalitischen Hydrierung unterwirft, um (+)-(1R,2R)-2,6,6-Trimethyl-bicyclo[3.1.1]heptan-3-spiro-1'-(4'-cyclohexanon) der Formel (Ib)

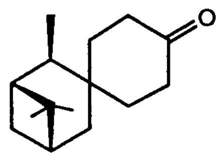

(Ib)

zu erhalten.